# EUROPEAN PATENT SPECIFICATION

(11) **EP 0 294 601 B1**
(45) Date of publication and mention of the grant of the patent: **20.01.1993**
(21) Application number: 88107473.6
(22) Date of filing: 10.05.1988
(51) Int. Cl.: A61K 31/44, A61K 47/14, A61K 9/06, A61K 47/16

(54) **Transdermal delivery of pharmaceuticals**
Transkutane Verabreichung von Pharmazeutika
Application transdermique de produits pharmaceutiques

(30) Priority: 12.06.1987 US 61842
(43) Date of publication of application: 14.12.1988
(73) Proprietor: AMERICAN CYANAMID COMPANY, Stamford Connecticut 06904-0060 (US)
(72) Inventor: Pawelchak, John Michael, Congers New York 10920 (US); Lawter, James Ronald, Goshen New York 10924 (US)
(74) Representative: Wächtershäuser, Günter, Prof. Dr.

(56) References cited:
- EP-A- 0 131 228
- EP-A- 0 164 588
- DE-A- 2 940 833
- US-A- 4 284 634
- US-A- 4 338 322
- PATENT ABSTRACTS OF JAPAN, vol. 10, no. 131 (C-346)(2188), 15th May 1986
- J. CARDIOVASC. PHARMACOL., vol. 8, no. 2, 1986, pages 221-226; HOF, R.P. et al.: "Stereoselectivity at the cacium channel: different profiles of hemodynamic activity of the Enantiomers of the Dihydropyridine Derivative PN 200-110."

## Description

This invention is concerned with the transdermal administration of the enantiomers of active enantiomers of pharmacologically active chiral compounds.

A transdermal delivery system is a pharmaceutical composition of matter which is applied to the skin in order to deliver the pharmaceutical through the skin to achieve a systemic therapeutic effect as distinguished from a local therapeutic effect.

If a drug exhibits transdermal fluxes that are too low to provide therapeutic plasma concentrations, an enhancer may be utilized to increase the transdermal flux. The flux enhancer is a substance, usually a solvent or vehicle that is applied to the skin in combination with a drug to increase the transdermal flux of the drug. Enhancers are believed to function by disrupting the barrier of the skin or by changing the partitioning behavior of the drug in the skin.

With or without an enhancer, the rate of transdermal flux determines whether percutaneous administration will provide sufficient drug absorption to achieve therapeutic plasma concentrations. This rate is a function of the drug's molecular diffusivity, partition coefficient and solubility in the skin.

The applicants have discovered that certain pharmacologically active chiral compounds may be administered transdermally as the resolved pure enantiomer or as a enantiomeric mixture containing a disproportionate amount of one enantiomer and substantially higher fluxes will be observed than with the transdermal administration of a racemic modification of the same compound using the same transdermal delivery system. It will be understood when reference is made to the enantiomer it is meant also include the substantially pure enantiomer which may contain very small amounts of the other enantiomer.

Accordingly, it is a primary object of the invention to provide compositions of an enantiomer of a pharmacologically active chiral compound or an enantiomeric mixture containing a disproportionate mixture of one enantiomer of a pharmacologically active chiral compound and a flux enhancer that will provide high flux when applied to the skin.

It is also an object of the invention to provide the use of pharmacologically active compositions which contain an enantiomer or an enantiomeric mixture containing a disproportionate amount of a pharmacologically active chiral compound for the manufacture of a medicament for transdermal administration.

As used herein and in the appended claims, the term enantiomeric mixture is used to define a mixture containing two enantiomers in any proportion. The term racemic modification is used to define an assembly of chiral molecules one-half of which are mirror images of the other. In the solid state, enantiomeric mixtures may be classified as (1) a racemic conglomerate, (2) a racemic compound or (3) a solid solution. This classification appears in H.W.B. Roozeboom, Z. Physik. Chem. 28, 494 (1899); H. Mariser, Chem. Ber. 90,307 (1957) and Eliel, E.L., Stereochemistry of Carbon Compounds. McGraw Hill Book Co., Inc. NY 1962, Chapter 4, pp. 31-86. The term disproportionate mixture of enantiomers is used to include enantiomeric mixtures having other than 50:50 ratio of (+) and (-) enantiomer. The preferred disproportionate mixtures have about 55:45 and 45:55 parts by weight of the (+) and (-) enantiomer.
The invention provides a transdermal delivery system which comprises:
(a) an amount of a substantially pure enantiomer or an enantiomer mixture having other than 50 : 50 ratio (+) and (-) enantiomers of a pharmalogically active chiral compound wherein the racemic modification of said pharmalogically active compound is solid at or above 37°C and has a melting point that is 5°C above the melting point of a 55 : 45 to 45 : 55 enantiomer mixture; and
(b) a vehicle for said pharmalogically active chiral compound.

### DETAILED DESCRIPTION OF THE INVENTION

The applicants have discovered that compositions containing a minor amount of certain pharmacologically active enantiomers of pharmacologically active chiral compounds may be administered transdermally and the flux when applied to the skin will be higher than the absorption rate obtained by transdermal administration of the racemic modification.

The pharmacologically active compounds within the scope of the invention are those for which the racemic modification is solid at or above 37°C. and wherein the racemic modification has a higher melting point than an enantiomer or an enantiomeric mixture containing a disproportionate amount of enantiomers.

Generally, the racemic modification should have a melting point that is at least 5-10°C. higher than the melting point of the enantiomer or any enantiomeric mixture containing a disproportionate amount of enantiomers. The melting point determination may be determined according to Wilen et al., Enantiomers Racemates and Resolutions, John Wiley & Sons, New York (1981).

In certain cases it may be advantageous to select a enantiomeric mixture containing a disproportionate amount of enantiomers that exhibit a eutectic melting point. This is advantageous because the lower melting composition will exhibit increased transdermal flux.

Suitable compounds that may be utilized in the practice of the invention include ephedrine, 3-hydroxy-N-methyl morphinan, propoxyphene, 1,4-dihydropyridine chiral compounds and in particular compounds of the formula: 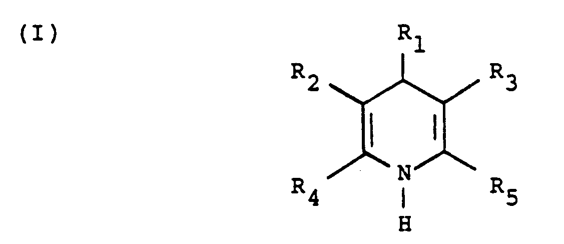
in the form of a pharmacologically active enantiomer wherein
R₁ is aryl which may have one or more suitable substituents(s) or a heterocyclic group,
R₂ and R₃ are each, same or different, esterified carboxy, and
R₄ and R₅ are each hydrogen, cyano, lower alkyl, or substituted lower alkyl in which the substituent is cyano, hydroxy, acyloxy, hydroxyimino, hydrazino, lower alkoxyimino, hydroxy(lower)alkylimino, N′ -or N′,N′-di(lower)alkylamino(lower)alkylimino, hydrazino, hydroxy(lower)alkylamino, N′- or N′,N′-di(lower)alkylamino(lower)alkylamino, a 5 or 6-membered saturated N-containing heterocyclic-lyl which may have hydroxy, lower alkyl or hydroxy(lower)alkyl, or oxo wherein the thus formed carbonyl may be protected with suitable protecting group; provided that, when one of R₄ and R₅ is hydrogen or lower alkyl the other is always cyano or said substituted lower alkyl, and when R₄ and R₅ are not hydrogen or lower alkyl, both of them are a group selected from cyano and said substituted lower alkyl,
or R₄ is hydrogen or lower alkyl and R₃ and R₅ are combined to form a group of the formula: 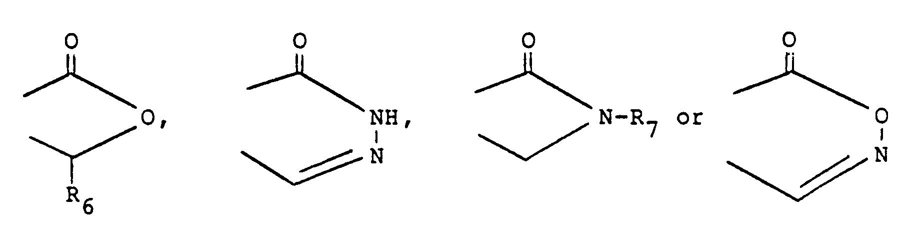
wherein R₆ is hydrogen or methyl and R₇ is 2-(N,N-diethylamino)ethyl or 2-hydroxyethyl.

The terms used in the definitions of the symbols of the general formulae given in this specification and claims are explained as follows:

The term "lower" used in connection with an alkylene, alkyl and alkenyl is intended to mean the one having 1 or 2 to 8 carbon atoms.

The aryl and aryl moieties may be phenyl, naphthyl, xylyl, tolyl, mesityl and cumenyl, which may have one or more suitable substituent(s). Preferred examples of the suitable substituent(s) are halogen, nitro, hydroxy, halo(lower)-alkyl, lower alkoxy, lower alkenyloxy, cyano, lower alkoxycarbonyl or lower alkylsulfamoyl. The halogen or halo moieties are fluorine, chlorine, bromine or iodine.

Lower alkylene moieties may have a straight or branched and saturated bivalent hydrocarbon chain such as methylene, ethylene, methylmethylene, trimethylene, propylene or tetramethylene.

Lower alkyl and lower alkyl moieties may have a straight or branched and saturated hydrocarbon chain such as methyl, ethyl, propyl, isopropyl, butyl, isobutyl, t-butyl, pentyl, neo-pentyl, hexyl, heptyl or octyl.

Lower alkoxy and lower alkoxy moieties may be methoxy, ethoxy, propoxy, isopropoxy, butyoxy, t-butoxy and pentyloxy.

Halo(lower)alkyl moieties may be mono-halo(lower)alkyl such as chloromethyl, bromomethyl or chloropropyl; dihalo(lower alkyl such as 1,2-dichloroethyl, 1,2-dibromoethyl or 2,2-dichloroethyl; and tri-halo(lower)alkyl such as trifluoromethyl or 1,2,2,-trichloroethyl.

Lower alkenyl and lower alkenyl moieties may be ones having a straight or branched hydrocarbon chain which contains one or more double bond(s), such as vinyl, allyl, butenyl, butanedienyl or penta-2,4-dienyl.

Acyl and acyl moieties may be lower alkanoyl such as formyl, acetyl, propionyl, butyryl, isobutyryl, valeryl, isovaleryl, pivaloyl; substituted lower alkanoyl, for example, carboxy(lower)-alkanoyl, esterified carboxy(lower)-alkanoyl such as lower alkoxycarbonyl(lower)alkanoyl, Nor N,N-di-substituted amino(lower)alkanoyl such as Nor N,N-di(lower)alkylamino(lower)alkanoyl (e.g. N-methyl-(or N,N-diethyl) aminoacetyl, 1(or2)-[N-ethyl(or N,N-diethyl)amino]proprionyl or 1 (or 2)-[N-methyl-N-ethylamino]propionyl) or N-lower alkyl-N-ar(lower)alkylamino(lower)alkanoyl (e.g. 1-(or 2)-[N-methyl-N-benzylamino]propionyl) or aryloxy(lower)alkanoyl such as phenoxyacetyl, tolyloxyacetyl, 2(or 3 or 4)-chlorophenoxyacetyl, 2-[2(or 3 or 4)-chlorophenoxy]propionyl, 2(or 3 or 4)-nitrophenoxyacetyl or 2(or 3 or 4)-methoxyphenoxyacetyl); aroyl such as benzoyl, naphthoyl or toluoyl.

Lower alkoxycarbonyl moieties may be methoxycarbonyl, ethoxycarbonyl, propoxycarbonyl, isopropoxycarbonyl, butoxycarbonyl and t-butoxycarbonyl.

Lower alkylsulfamoyl moieties may be methylsulfamoyl, ethylsulfamoyl, propylsulfamoyl, isopropylsulfamoyl, butylsulfamoyl and pentylsulfamoyl.

A heterocyclic group designated R₁ may be an aromatic heterocyclic group containing one or more hetero atom(s) selected form a nitrogen atom, a sulfur atom and an oxygen atom, for example, thienyl, furyl, pyrrolyl, thiazolyl, thiadiazolyl, tetrazolyl, pyridyl, pyrimidinyl, quinolyl, isoquinolyl, benzothienyl, indolyl or purinyl.

Esterifed carboxy groups designated R₂ and R₃ may be lower alkoxycarbonyl such as methoxycarbonyl, ethoxycarbonyl, propoxycarbonyl, butoxycarbonyl, t-butoxycarbonyl; halo(lower)alkoxycarbonyl such as the haloanalogues of the above-mentioned lower alkoxycarbonyl (e.g., 2-bromoethoxycarbonyl, 2-chloroethoxycarbonyl, 2(or 3)-chloropropoxycarbonyl, 2 (or 3)-bromopropoxycarbonyl, 2,2-dichloroethoxycarbonyl or 2,2,2-trichroloethoxycarbonyl); hydroxy(lower)-alkoxycarbonyl such as 2-hydroxyethoxycarbonyl or 2(or 3)-hydroxypropoxycarbonyl; lower alkoxy(lower)alkoxycarbonyl such as 2-methoxyethoxycarbonyl, 2-ethoxyethoxycarbonyl or 2(or 3)-methoxy(or ethoxy)-propoxycarbonyl; aryloxycarbonyl such as phenoxycarbonyl, tolyloxycarbonyl, xylyloxycarbonyl or p-chlorophenoxycarbonyl; ar(lower)alkoxycarbonyl such as benzyloxycarbonyl, p-bromobenzyloxycarbonyl, 0-methoxybenzyloxycarbonyl or phenethyloxycarbonyl; ar(lower)alkoxy(lower)-alkoxycarbonyl such as 2-(benzyloxyl)ethoxycarbonyl or 2(or 3)-(benzyloxy)propoxycarbonyl; aryloxy(lower)alkoxycarbonyl such as 2-(phenoxy)ethoxycarbonyl or 2(or 3)-(phenoxy)propoxycarbonyl; Nor N,N-(di)-substituted amino(lower)-alkoxycarbonyl such as Nor N,N-(di)-(lower)-alkylamino(lower) alkoxycarbonyl (e.g., 1(or 2)-[N-methyl(or N,N-dimethyl)amino]ethoxycarbonyl, 1(or2)-[N-ethyl(or N,N-diethyl)amino]ethoxycarbonyl, or 1(or 2)-N-methyl-N-ethylamino)ethoxycarbonyl or lower alkyl-N-ar(lower)alkylamino(lower)alkoxycarbonyl (e.g. 2-(N-methyl-N-benzylamino)ethoxycarbonyl), and further R₂ and R₃ may be same or different.

Lower alkyl substituted with oxo includes lower alkanoyl such as formyl, acetyl, propionyl, butyryl, isobutyryl, valeryl, isovaleryl or pivaloyl and lower alkanoyl(lower)alkyl such as formylmethyl, acetonyl, 2-formylethyl, 3-formylpropyl or butyrylmethyl. The carbonyl group thereof may be protected with suitable protecting group, and thus protected carbonyl group in this invention means a group given by protecting the carbonyl with conventionally employed protecting group for a carbonyl. Suitable examples of such protected carbonyl groups are acetal, cyclic-acetal, thioacetal, cyclic-thioacetal, cyclicmonothioacetal or acylal types of group. Examples of these lower alkyl groups containing such protected carbonyl group are gen-di-(lower)-alkoxy(lower)alkyl (e.g. dimethoxymethyl, 1,1-dimethoxyethyl, diethoxymethyl, dipropoxymethyl, 2,2-diethoxyethyl or 2,2-diethoxypropyl; gem-lower alkylenedioxy(lower)alkyl (e.g. 1,3-dioxolan-2-yl, 2-methyl-1,3-dioxolan-2-yl, 4-methyl-1,3-dioxolan-2-yl, 4,5-dimethyl-1,3-dioxolan-2-yl, 1,3-dioxan-2-yl, 2-methyl-1,3-dioxan-2-yl, 1,3-dioxolan-2-yl-methyl, 2-methyl-1,3-dioxolan-2-yl-methyl or 3-(1,3-dioxolan-2-yl)propyl); gem-di-(lower)alkylthio(lower)-alkyl (e.g., dimethylthiomethyl, 1,1-dimethylthioethyl, diethylthiomethyl or 2,2-diethylthioethyl); gem-lower alkylenedithio(lower)alkyl (e.g. 1,3-dithiolan-2-yl, 2-methyl-1,3-dithiolan-2-yl, 4-methyl-1,3-dithiolan-2-yl, 4,5-dimethyl-1,3-dithiolan-2-yl, 1,3-dithian-2-yl, 2-methyl-1,3-dithian-2-yl, 1,3-dithiolan-2-yl-methyl, 2-methyl-1,3-dithiolan-2-ylmethyl or 3-(1,3-dithiolan-2yl)propyl); and gem-di(lower)alkanoyloxy(lower)-alkyl (e.g., diacetoxymethyl, 1,1-diacetoxyethyl, dipropionyloxymethyl or 2,2-dipropionyloxyethyl); 5 or 6-membered saturated 1-oxa-3-thioheterocyclic-1-yl-(lower)alkyl (e.g., 1,3-oxathiolan-2-yl, 2-methyl-1,3-oxathiolan-2-yl, 4-methyl-1,3-oxathiolan-2-yl, 4,5-dimethyl-1,3-oxathiolan-2-yl, 1,3-oxothian-2-yl, 2-methyl-1,3-oxothian-2-yl, 1,3-oxathiolan-2-ylmethyl, 2-methyl-1,3-oxathiolan2-ylmethyl or 3-(1,3-oxathiolan-2-yl)propyl).

A 5 or 6-membered saturated N-containing heterocyclic-1-yl) group may be one which may contain additional one or more hetero atom(s) selected from nitrogen, sulfur and oxygen atoms such as pyrrolidin-1-yl, piperidino, imidazolidin-1-yl, morpholino or thiomorpholino, and it may be optionally substituted with hydroxy, lower alkyl or hydroxy(lower)alkyl such as hydroxymethyl, 2-hydroxyethyl, 2-hydroxypropyl or 3-hydroxypropyl.

The other terms of each lower alkoxyimino, N′- or N′,N′-di-(lower)alkylamino(lower)alkylimino, hydroxy(lower)alkylamino, N′- or N′,N′-di(lower)alkylamino(lower)alkylamino and hydroxy(lower)alkylamino will be clearly defined by applying optionally the above given exemplifications of the terms to them.

The (+) or (-) enantiomers may be obtained using standard procedures such as by chiral chromatography of the racemate or by other means.

The preferred compound is nilvadipine.

The compositions of the invention may be prepared by admixing the flux enhancer with the drug. The concentration of the drug will depend on the particular drug and the particular enhancer. Generally, solutions of up to and including saturated solutions of the drug may be employed. In addition saturated solutions which contain up to 50% dispersed, undissolved drug may be used. If desired the flux enhancing agent and drug may be placed in a transdermal patch. In addition other ingredients such as gelling agents; e.g. hydroxypropyl cellulose; viscous adhesive agents; polymeric additives, e.g. thickeners; processing agents; stabilizers; preservatives; UV absorbers; antioxidants; viscosity increasing agents may be added.

Suitable vehicles for use in the transdermal administration of compounds of the invention may be selected by those who are skilled in the art. If desired, a flux enhancer may be used to achieve therapeutic levels of the drug. In the case of the above-mentioned 1,4-dihydropyridines, suitable flux enhancers may be selected from the group consisting of esters of C₁₂ C₁₈ fatty acids with C₁ - C₆ straight and branched chain alcohols; diesters of aliphatic diacids of the formula:

R₈ OOC (CH₂)ₙ COO R₉

wherein n is a whole integer from 2-8; R₈ and R₉ may be the same or different and are selected from the group consisting of C₂ to C₁₂ straight and branched chain alcohols; and compounds of the formula: 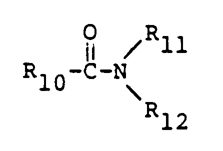
wherein R₁₀ is a C₇ - C₁₃ straight or branched chain alkyl or alkenyl group R₁₁ and R₁₂ are the same or different and are selected from -CH₂CH₂OH and -CH₂CHOHCH₃ and hydrogen; benzyl alcohol, 2 phenylethanol, ethanol or mixtures thereof with ethyl alcohol.

The C₁₂ - C₁₈ fatty acids include lauric, tridecanoic, myristic, pentadecanoic, palmitic, margaric and stearic. The straight and branched chain alcohols include methanol, ethanol, n-propanol, i-propanol, n-pentanol and n-hexanol.

The diesters include the diesters of succinic, glutaric, adipic, pimelic, suberic, azelaic and sebacic acids with ethanol, n-propanol, i-propanol, n-hexanol and n-octanol.

Compounds of the formula: 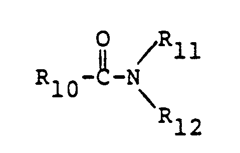
include those where R₁₀ is capryl, undecanyl, lauryl 3-octenyl, 4-decenyl, 9-decenyl or tridecanyl and R₁₁ and R₁₂ are ethylhydroxy or propylhydroxy. The compound N,N-diethylhydroxy lauramide is preferred.

Ethanol may be employed at levels that are sufficient to modify the flux. It may comprise from 10-95% of the total flux enhancer. Light mineral oil or silicone may be employed to control the thermodynomic activity of the flux enhancers. Useful silicones include polydimethyl polysiloxones. The amount of such materials may be varied to change the rate of absorption of a drug from a particular flux enhancer.

Illustratively, the composition can be provided and used which comprises nilvadipine, 0.01 to 50% by weight with the balance of the composition comprising the flux enhancer alone or in combination with other additives.

### DESCRIPTION OF THE PREFERRED EMBODIMENT

The following example illustrates the present invention.

### EXAMPLE

By way of illustration the following table shows the solubility in mg/ml at 32°C of the racemic modification and the (+) enantiomer of nilvadipine in different solvents.

**Table 1**

| | Polyethylene glycol 400 (30% w/w in H₂O) | Ethanol | Diisopropyl Adipate | 10% w/w Diisopropyl Adipate in Ethanol |
|---|---|---|---|---|
| nilvadipine (±) | 0.08 | 38 | 58 | 56 |
| nilvadipine (+) | 0.115 | 170 | 142 | 560 |

The transdermal penetration rate of the racemic modification and the (+) enantiomer of nilvadipine was determined by determining the permeation rate through split-thickness human cadaver skin.

The cadaver skin is placed in a nutrient medium (Dulbecco's Minimum Essential Medium containing a mycostat and a bacteriostat) on a collagen pad. A disc of skin about 2cm in diameter is cut and placed in a Bronough transdermal transport cell at 32°C. The dermal surface is contacted with a receptor fluid that consists of polyethylene glycol 400 as a 30% w/w solution in water. The solution of the drug is placed on the skin with a dropper and the flux value is read after steady state conditions have been reached. A carbon-14 radiolabeled drug and a calibrated scintillation counter is used to determine the amount of drug transported.

The permeation rate through split-thickness human cadaver skin of the solution set forth in Table I are reported in Table II in terms of nanograms/cm²/hour.

**Table II**

| Vehicle | Racemic Modification | Enantiomer |
|---|---|---|
| 30% w/w PEG 400 in water | 5 | 28 |
| Diisopropyl Adipate | 360 | 850 |
| Ethanol | 150 | 560 |

The results set forth in Table II show that solutions of the enantiomer of nilvadipine are more rapidly absorbed than solutions of the racemic modification.

The compositions of the invention may be placed in conventional transdermal delivery systems for administration to a host.

## Claims (Claims for the following Contracting State(s): AT, BE, CH, DE, FR, GB, IT, LI, NL, SE)

1. A transdermal delivery system which comprises:
(a) an amount of a substantially pure enantiomer or an enantiomer mixture having other than 50 : 50 ratio (+) and (-) enantiomers of a pharmalogically active chiral compound wherein the racemic modification of said pharmalogically active compound is solid at or above 37°C and has a melting point that is 5°C above the melting point of a 55 : 45 to 45 : 55 enantiomer mixture; and
(b) a vehicle for said pharmalogically active chiral compound.

2. A transdermal delivery system as defined in Claim 1, wherein the substantially pure enantiomer is employed.

3. A transdermal delivery system as defined in Claim 1, wherein an enantiomer mixture having other than 50 : 50 ratio of (+) and (-) enantiomers is employed that form an eutectic.

4. A transdermal delivery system as defined in Claim 2 wherein the enantiomer of the pharmacologically active chiral compound is selected from compounds of the formula: 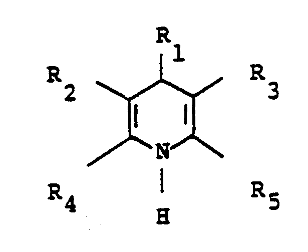 and a resolved enantiomer thereof, wherein
R₁ is aryl which may have one or more suitable substituents(s) or a heterocyclic group,
R₂ and R₃ are each, same or different, esterified carboxy, and
R₄ and R₅ are each hydrogen; cyano; lower alkyl; or substituted lower alkyl in which the substituent is cyano, hydroxy, acyloxy, hydroxyimino, hydrazino, C₁₋₈ alkoxyimino, hydroxy (C₁₋₈) alkylimino, N'- or N',N'-di(C₁₋₈) alkylamino (C₁₋₈) alkylimino, hydrazino, hydroxy(C₁₋₈) alkylamino, N'- or N',N'-di(C₁₋₈) alkylamino (C₁₋₈) alkylamino, a 5 or 6-memebered saturated N-containing heterocyclic-lyl which may have hydroxy, C₁₋₈ alkyl or hydroxy (C₁₋₈) alkyl, or oxo wherein the thus formed carbonyl may be protected with suitable protecting group; provided that, when one of R₄ and R₅ is hydrogen or C₁₋₈ alkyl, the other is always cyano or said substituted C₁₋₈ alkyl, both of them are selected from cyano and said substituted lower alkyl,
or R₄ is hydrogen or C₁₋₈ alkyl and R₃ and R₅ are combined to form a group of the formula: 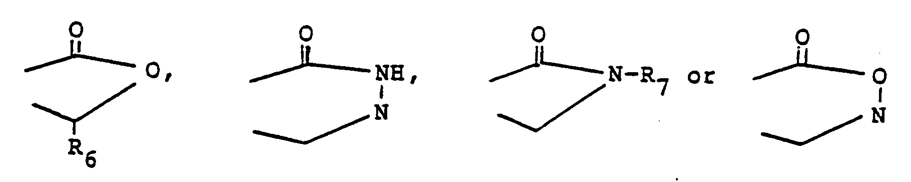 wherein R₆ is hydrogen or methyl and R₇ is 2-(N,N-diethylamino)-ethyl or 2-hydroxyethyl, wherein the term "lower" means 1 or 2 to 8 carbon atoms.

5. A transdermal delivery system as defined in Claim 3 wherein the enantiomer of the pharmacologically active chiral compound is (+) nilvadipine.

6. A transdermal delivery system as defined in Claim 5 wherein the vehicle comprises a flux enhancer selected from esters of C₁₂ - C₁₈ fatty acid esters with C₁ - C₆ straight and branched chain alcohols; diesters of diacids of the formula:
R₈ OOC (CH₂)ₙ COO R₉
wherein n is an integer of 2-8, R₈ and R₉ may be the same or different and are selected from C₂ - C₁₂ straight and branched chain alcohols; compounds of the formula: 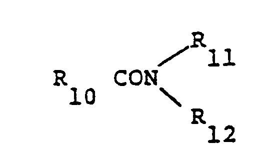 wherein R₁₀ is a straight or branched chain residue of a C₈ - C₁₄ fatty acid, R₁₁ and R₁₂ are the same or different and are the same or different and are selected from -CH₂CH₂OH and -CH₂CH₂CH₂OH; benzyl alcohol, 2 phenylethanol, ethanol or mixtures of said solvents with 10-90% ethanol.

7. A transdermal delivery system as defined in Claim 5 wherein the solvent is diisopropyl adipate.

## Claims (Claims for the following Contracting State(s): ES, GR)

1. A method of preparing a transdermal delivery system which comprises combining:
(a) an amount of a substantially pure enantiomer or an enantiomer mixture having other than 50 : 50 ratio (+) and (-) enantiomers of a pharmalogically active chiral compound wherein the racemic modification of said pharmalogically active compound is solid at or above 37°C and has a melting point that is 5°C above the melting point of a 55 : 45 to 45 : 55 enantiomer mixture; and
(b) a vehicle for said pharmalogically active chiral compound.

2. A method as defined in Claim 1, wherein the substantially pure enantiomer is employed.

3. A method as defined in Claim 1, wherein an enantiomer mixture having other than 50 : 50 ratio of (+) and (-) enantiomers is employed that form an eutectic.

4. A method as defined in Claim 2 wherein the enantiomer of the pharmacologically active chiral compound is selected from compounds of the formula: 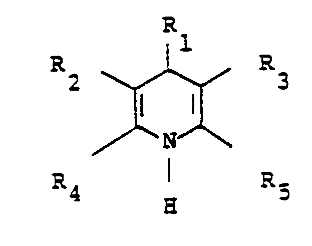 and a resolved enantiomer thereof, wherein
R₁ is aryl which may have one or more suitable substituents(s) or a heterocyclic group,
R₂ and R₃ are each, same or different, esterified carboxy, and
R₄ and R₅ are each hydrogen; cyano; lower alkyl; or substituted lower alkyl in which the substituent is cyano, hydroxy, acyloxy, hydroxyimino, hydrazino, C₁₋₈ alkoxyimino, hydroxy (C₁₋₈) alkylimino, N'- or N',N'-di(C₁₋₈) alkylamino (C₁₋₈) alkylimino, hydrazino, hydroxy(C₁₋₈) alkylamino, N'- or N',N'-di(C₁₋₈) alkylamino (C₁₋₈) alkylamino, a 5 or 6-memebered saturated N-containing heterocyclic-lyl which may have hydroxy, C₁₋₈ alkyl or hydroxy (C₁₋₈) alkyl, or oxo wherein the thus formed carbonyl may be protected with suitable protecting group; provided that, when one of R₄ and R₅ is hydrogen or C₁₋₈ alkyl, the other is always cyano or said substituted C₁₋₈ alkyl, both of them are selected from cyano and said substituted lower alkyl,
or R₄ is hydrogen or C₁₋₈ alkyl and R₃ and R₅ are combined to form a group of the formula: 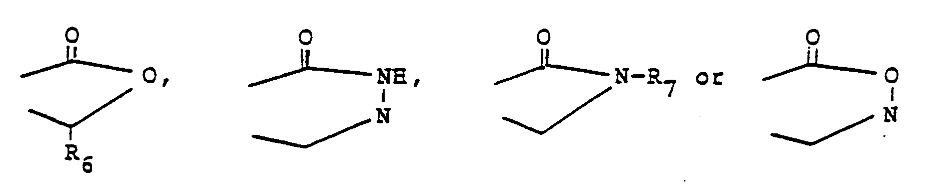 wherein R₆ is hydrogen or methyl and R₇ is 2-(N,N-diethyl-amino)ethyl or 2-hydroxyethyl, wherein the term "lower" means 1 or 2 to 8 carbon atoms.

5. A method as defined in Claim 3 wherein the enantiomer of the pharmacologically active chiral compound is (+) nilvadipine.

6. A method as defined in Claim 5 wherein the vehicle comprises a flux enhancer selected from esters of C₁₂ - C₁₈ fatty acid esters with C₁ - C₆ straight and branched chain alcohols; diesters of diacids of the formula:
R₈ OOC (CH₂)ₙCOO R₉
wherein n is integer of 2-8, R₈ and R₉ may be the same or different and are selected from C₂ - C₁₂ straight and branched chain alcohols; compounds of the formula: 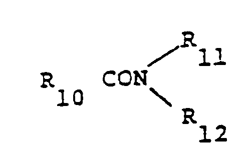 wherein R₁₀ is a straight or branched chain residue of a C₈ - C₁₄ fatty acid, R₁₁ and R₁₂ are the same or different and are the same or different and are selected from -CH₂CH₂OH and -CH₂CH₂CH₂OH; benzyl alcohol, 2 phenylethanol, ethanol or mixtures of said solvents with 10-90% ethanol.

7. A method as defined in Claim 5 wherein the solvent is diisopropyl adipate.

## Patentansprüche (Patentansprüche für folgende(n) Vertragsstaat(en): AT, BE, CH, DE, FR, GB, IT, LI, NL, SE)

1. Transdermales Abgabesystem, umfassend:
(a) eine Menge eines im wesentlichen reinen Enantiomeren oder einer Enantiomerenmischung mit einem vom 50 : 50 abweichenden Verhältnis der (+)- und (-)-Enantiomeren einer pharmakologisch aktiven chiralen Verbindung, wobei die racemische Modifikation der erwähnten pharmakologisch aktiven Verbindung bei oder über 37 °C fest ist und einen Schmelzpunkt aufweist, der 5 °C höher ist als der Schmelzpunkt einer 55 : 45 zu 45 : 55 Enantiomerenmischung; und
(b) ein Vehikel für die erwähnte pharmakologisch aktive chirale Verbindung.

2. Transdermales Abgabesystem, wie es in Anspruch 1 definiert ist, wobei das im wesentlichen reine Enantiomere eingesetzt wird.

3. Transdermales Abgabesystem, wie es in Anspruch 1 definiert ist, wobei eine Enantiomerenmischung mit einem von 50 : 50 abweichenden Verhältnis der (+)- und (-)-Enantiomeren eingesetzt wird, die ein Eutektikum bildet.

4. Transdermales Abgabesystem, wie es in Anspruch 2 definiert ist, wobei das Enantiomere der pharmakologisch aktiven chiralen Verbindung ausgewählt ist aus Verbindungen der Formel: 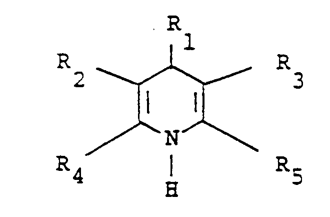 und einem abgetrennten Enantiomeren derselben, wobei
R₁ für Aryl, das ein oder mehrere geeignete Substituenten aufweisen kann, oder eine heterocyclische Gruppe steht,
R₂ und R₃, die jeweils gleich oder verschieden sind, für verestertes Carboxy stehen, und
R₄ und R₅ jeweils für Wasserstoff, Cyano, Niederalkyl oder substituiertes Niederalkyl, wobei der Substituent Cyano, Hydroxy, Acyloxy, Hydroxyimino, Hydrazin, C₁₋₈-Alkoxyimino, Hydroxy-(C₁₋₈)-alkylimino, N'- oder N',N'-Di-(C₁₋₈)alkylamino-(C₁₋₈)-alkylimino, Hydrazino, Hydroxy-(C₁₋₈)-akylamino, N'- oder N',N'-Di-(C₁₋₈)-alkylamino-(C₁₋₈)-alkylamino ist, steht, oder für einen 5- oder 6-gliedrigen, gesättigen N-haltigen Heterocyclus, der Hydroxy, C₁₋₈-Alkyl oder Hydroxy-(C₁₋₈)-alkyl aufweisen kann, oder für Oxo steht, wobei das so gebildete Carbonyl geschützt sein kann mit einer geeigneten Schutzgruppe, mit der Maßgabe, daß dann, wenn R₄ und R₅ für Wasserstoff oder C₁₋₈-Alkyl steht, der andere Rest immer für Cyano oder das erwähnte substituierte C₁₋₈-Alkyl steht, wobei diese beiden ausgewählt sind aus Cyano und dem erwähnten substituierten Niederalkyl,
oder R₄ für Wasserstoff oder C₁₋₈-Alkyl steht und R₃ und R₅ kombiniert sind unter Bildung einer Gruppe der Formel: 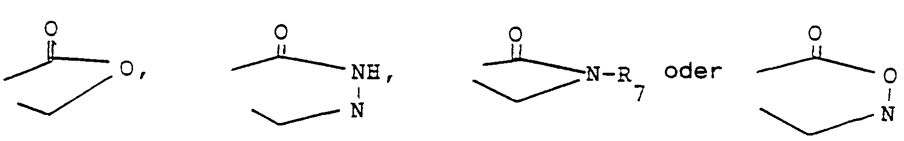 wobei R₆ für Wasserstoff oder Methyl steht und R₇ für 2-(N,N-Diethylamino)-ethyl oder 2-Hydroxyethyl steht, wobei der Ausdruck "nieder" 1 oder 2 bis 8 Kohlenstoffatome bedeutet.

5. Transdermales Abgabesystem, wie es in Anspruch 3 definiert ist, wobei das Enantiomere der pharmakologisch aktiven chiralen Verbindung (+)-Nilvadipin ist.

6. Transdermales Abgabesystem, wie es in Anspruch definiert ist, wobei das Vehikel ein Mittel zur Verbesserung des Fließens umfaßt, ausgewählt aus Estern von C₁₂-C₁₈-Fettsäureestern mit C₁₋₆-geradkettigen und verzweigtkettigen Alkoholen; Diestern von Disäuren der Formel:
R₈-OOC-(CH₂)n-COO-R₉ ,
wobei n für eine ganze Zahl von 2 bis 8 steht, R₈ und R₉ gleich oder verschieden sein können und ausgewählt sind aus C₂₋₁₂-geradkettigen und verzweigtkettigen Alkoholen; Verbindungen der Formel 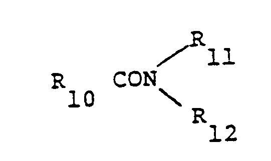 wobei R₁₀ für einen geradkettigen oder verzweigtkettigen Rest einer C₈₋₁₄-Fettsäure steht, R₁₁ und R₁₂ jeweils gleich oder verschieden sind und ausgewählt sind aus -CH₂CH₂OH und -CH₂CH₂CH₂OH; Benzylalkohol, 2-Phenylethanol, Ethanol oder Mischungen der erwähnten Lösungsmittel mit 10 bis 90 % Ethanol.

7. Transdermales Abgabesystem, wie es in Anspruch 5 definiert ist, wobei das Lösungsmittel Diisopropyladipat ist.

## Patentansprüche (Patentansprüche für folgende(n) Vertragsstaat(en): ES, GR)

1. Verfahren zur Herstellung eines transdermalen Abgabesystems, umfassend das Kombinieren von
(a) eine Menge eines im wesentlichen reinen Enantiomeren oder einer Enantiomerenmischung mit einem vom 50 : 50 abweichenden Verhältnis der (+)- und (-)-Enantiomeren einer pharmakologisch aktiven chiralen Verbindung, wobei die racemische Modifikation der erwähnten pharmakologisch aktiven Verbindung bei oder über 37 °C fest ist und einen Schmelzpunkt aufweist, der 5 °C höher ist als der Schmelzpunkt einer 55 : 45 zu 45 : 55 Enantiomerenmischung; und
(b) ein Vehikel für die erwähnte pharmakologisch aktive chirale Verbindung.

2. Ein Verfahren, wie es in Anspruch 1 definiert ist, wobei das im wesentlichen reine Enantiomere eingesetzt wird.

3. Ein Verfahren, wie es in Anspruch 1 definiert ist, wobei eine Enantiomerenmischung mit einem von 50 : 50 abweichenden Verhältnis der (+)- und (-)-Enantiomeren eingesetzt wird, die ein Eutektikum bildet.

4. Ein Verfahren, wie es in Anspruch 2 definiert ist, wobei das Enantiomere der pharmakologisch aktiven chiralen Verbindung ausgewählt ist aus Verbindungen der Formel: 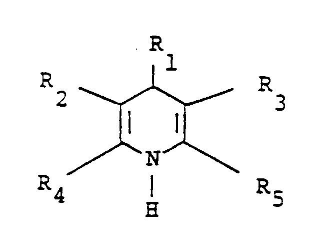 und einem abgetrennten Enantiomeren derselben, wobei
R₁ für Aryl, das ein oder mehrere geeignete Substituenten aufweisen kann, oder eine heterocyclische Gruppe steht,
R₂ und R₃, die jeweils gleich oder verschieden sind, für verestertes Carboxy stehen, und
R₄ und R₅ jeweils für Wasserstoff, Cyano, Niederalkyl oder substituiertes Niederalkyl, wobei der Substituent Cyano, Hydroxy, Acyloxy, Hydroxyimino, Hydrazin, C₁₋₈-Alkoxyimino, Hydroxy-(C₁₋₈)-alkylimino, N'- oder N',N'-Di-(C₁₋₈)alkylamino-(C₁₋₈)-alkylimino, Hydrazino, Hydroxy-(C₁₋₈)-alkylamino, N'- oder N',N'-Di-(C₁₋₈)-alkylamino-(C₁₋₈)-alkylamino ist, steht, oder für einen 5- oder 6-gliedrigen, gesättigen N-haltigen Heterocyclus, der Hydroxy, C₁₋₈-Alkyl oder Hydroxy-(C₁₋₈)-alkyl aufweisen kann, oder für Oxo steht, wobei das so gebildete Carbonyl geschützt sein kann mit einer geeigneten Schutzgruppe, mit der Maßgabe, daß dann, wenn R₄ und R₅ für Wasserstoff oder C₁₋₈-Alkyl steht, der andere Rest immer für Cyano oder das erwähnte substituierte C₁₋₈-Alkyl steht, wobei diese beiden ausgewählt sind aus Cyano und dem erwähnten substituierten Niederalkyl,
oder R₄ für Wasserstoff oder C₁₋₈-Alkyl steht und R₃ und R₅ kombiniert sind unter Bildung einer Gruppe der Formel: 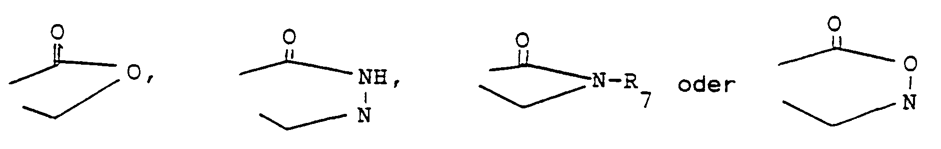 wobei R₆ für Wasserstoff oder Methyl steht und R₇ für 2-(N,N-Diethylamino)-ethyl oder 2-Hydroxyethyl steht, wobei der Ausdruck "nieder" 1 oder 2 bis 8 Kohlenstoffatome bedeutet.

5. Ein Verfahren, wie es in Anspruch 3 definiert ist, wobei das Enantiomere der pharmakologisch aktiven chiralen Verbindung (+)-Nilvadipin ist.

6. Ein Verfahren, wie es in Anspruch definiert ist, wobei das Vehikel ein Mittel zur Verbesserung des Fließens umfaßt, ausgewählt aus Estern von C₁₂₋₁₈-Fettsäureestern mit C₁₋₆-geradkettigen und verzweigtkettigen Alkoholen; Diestern von Disäuren der Formel:
R₈-OOC-(CH₂)n-COO-R₉,
wobei n für eine ganze Zahl von 2 bis 8 steht, R₈ und R₉ gleich oder verschieden sein können und ausgewählt sind aus C₂₋₁₂-geradkettigen und verzweigtkettigen Alkoholen; Verbindungen der Formel 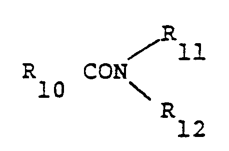 wobei R₁₀ für einen geradkettigen oder verzweigtkettigen Rest einer C₈₋₁₄-Fettsäure steht, R₁₁ und R₁₂ jeweils gleich oder verschieden sind und ausgewählt sind aus -CH₂CH₂OH und -CH₂CH₂CH₂OH; Benzylalkohol, 2-Phenylethanol, Ethanol oder Mischungen der erwähnten Lösungsmittel mit 10 bis 90 % Ethanol.

7. Ein Verfahren, wie es in Anspruch 5 definiert ist, wobei das Lösungsmittel Diisopropyladipat ist.

## Revendications (Revendications pour l'(les) Etat(s) contractant(s) suivant(s): AT, BE, CH, DE, FR, GB, IT, LI, NL, SE)

1. Un système d'apport transdermique qui comprend
(a) une quantité d'un énantiomère pratiquement pur ou d'un mélange d'énantiomères ayant un rapport autre que 50:50 des énantiomères (+) et (-) d'un composé chiral pharmacologiquement actif dans lequel la modification racémique dudit composé pharmacologiquement actif est solide à 37°C ou au-dessus et a un point de fusion qui est de 5°C au-dessus du point de fusion d'un mélange d'énantiomères 55:45 à 45:55 ; et
(b) un véhicule pour ledit composé chiral pharmacologiquement actif.

2. Un système tel que défini à la revendication 1, dans lequel on utilise l'énantiomère pratiquement pur.

3. Un système tel que défini à la revendication 1, dans lequel on utilise un mélange d'énantiomères ayant un rapport autre que 50:50 des énantiomères (+) et (-), qui forme un eutectique.

4. Un système d'apport transdermique tel que défini à la revendication 2, dans lequel l'énantiomère du composé chiral pharmacologiquement actif est choisi parmi les composés de formule : 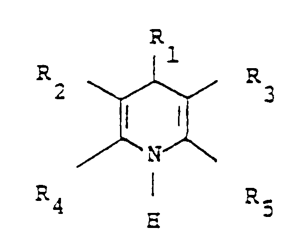 et leurs énantiomères séparés, dans laquelle
R₁ est un groupe aryle qui peut avoir un ou plusieurs substituants appropriés ou un groupe hétérocyclique,
R₂ et R₃ sont identiques ou différents et représentent chacun un groupe carboxy estérifié, et
R₄ et R₅ sont chacun un atome d'hydrogène ou un groupe cyano, alkyle inférieur ou alkyle inférieur substitué dans lequel le substituant est cyano, hydroxy, acyloxy, hydroxyimino, hydrazino, alcoxyimino en C₁-C₈, hydroxyalkylimino en C₁-C₈, N'- ou N',N'-di(alkyl en C₁-C₈)amino-(alkyl en C₁-C₈)imino, hydrazino, hydroxyalkylamino inférieur, N'- ou N',N'-di(alkyl en C₁-C₈)amino(alkyl en C₁-C₈)amino, un groupe hétérocyclique azoté saturé à 5 ou 6 chaînons qui peut avoir un groupe hydroxy, alkyle en C₁-C₈ ou hydroxyalkyle en C₁-C₈ ou oxo, dans lequel le groupe carbonyle ainsi formé peut être protégé par un groupement protecteur approprié ; pourvu que, lorsque l'un des restes R₄ et R₅ est l'hydrogène ou un groupe alkyle en C₁-C₈, l'autre soit toujours un groupe cyano ou ledit groupe alkyle en C₁-C₈ substitué et, lorsque R₄ et R₅ ne sont pas l'hydrogène ou un groupe alkyle inférieur, tous les deux soient choisis parmi un groupe cyano et ledit groupe alkyle inférieur substitué,
ou bien R₄ est un atome d'hydrogène ou un groupe alkyle en C₁-C₈ et R₃ et R₅ sont combinés pour former un groupe de formule : 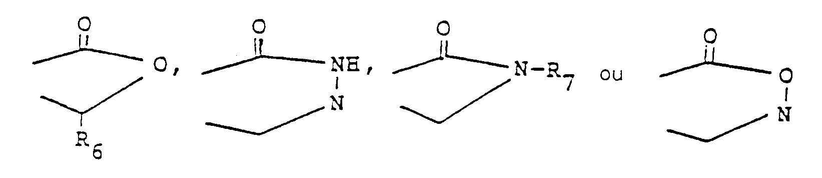 dans lesquelles R₆ est un atome d'hydrogène ou un groupe méthyle et R₇ est un groupe 2-(N,N-diéthylamino)éthyle ou 2-hydroxyéthyle, où le terme "inférieur" signifie 1 ou 2 à 8 atomes de carbone.

5. Un système d'apport transdermique tel que défini à la revendication 3, dans lequel l'énantiomère du composé chiral pharmacologiquement actif est la (+)-nilvadipine.

6. Un système d'apport transdermique tel que défini à la revendication 5, dans lequel le véhicule comprend un activateur de flux choisi parmi les esters d'acides gras en C₁₂-C₁₈ avec des alcools à chaîne droite ou ramifiée en C₁-C₆; les diesters de diacides de formule :
R₈OCC(CH₂)ₙ COO R₉
dans laquelle n est un entier de 2 à 8 et R₈ et R₉ peuvent être identiques ou différents et sont choisis parmi les alcools à chaîne droite et à chaîne ramifiée en C₂-C₁₂ ; les composés de formule : 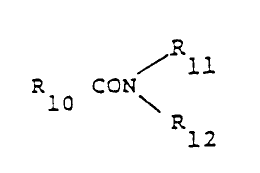 dans laquelle R₁₀ est un résidu à chaîne droite ou ramifiée d'un acide gras en C₈-C₁₄, R₁₁ et R₁₂ sont identiques ou différents et sont choisis parmi -CH₂CH₂OH et -CH₂CH₂CH₂OH ; l'alcool benzylique, le 2-phényléthanol, l'éthanol ou les mélanges de ces solvants avec l'éthanol à 10-90 %.

7. Un système d'apport transdermique tel que défini à la revendication 5, dans lequel le solvant est l'adipate de diisopropyle.

## Revendications (Revendications pour l'(les) Etat(s) contractant(s) suivant(s): ES, GR)

1. Un procédé de préparation d'un système d'apport transdermique qui consiste à combiner :
(a) une quantité d'un énantiomère pratiquement pur ou d'un mélange d'énantiomères ayant un rapport autre que 50:50 des énantiomères (+) et (-) d'un composé chiral pharmacologiquement actif dans lequel la modification racémique dudit composé pharmacologiquement actif est solide à 37°C ou au-dessus et a un point de fusion qui est de 5°C au-dessus du point de fusion d'un mélange d'énantiomères 55:45 à 45:55 ; et
(b) un véhicule pour ledit composé chiral pharmacologiquement actif.

2. Un procédé tel que défini à la revendication 1, dans lequel on utilise l'énantiomère pratiquement pur.

3. Un procédé tel que défini à la revendication 1, dans lequel on utilise un mélange d'énantiomères ayant un rapport autre que 50:50 des énantiomères (+) et (-), qui forme un eutectique.

4. Un procédé tel que défini à la revendication 2, dans lequel l'énantiomère du composé chiral pharmacologiquement actif est choisi parmi les composés de formule : 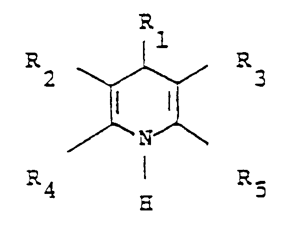 et leurs énantiomères séparés, dans laquelle
R₁ est un groupe aryle qui peut avoir un ou plusieurs substituants appropriés ou un groupe hétérocyclique,
R₂ et R₃ sont identiques ou différents et représentent chacun un groupe carboxy estérifié, et
R₄ et R₅ sont chacun un atome d'hydrogène ou un groupe cyano, alkyle inférieur ou alkyle inférieur substitué dans lequel le substituant est cyano, hydroxy, acyloxy, hydroxyimino, hydrazino, alcoxyimino en C₁-C₈, hydroxyalkylimino en C₁-C₈, N'- ou N',N'-di(alkyl en C₁-C₈)amino-(alkyl en C₁-C₈)imino, hydrazino, hydroxyalkylamino en C₁-C₈, N'- ou N',N'-di(alkyl en C₁-C₈)amino(alkyl en C₁-C₈)amino, un groupe hétérocyclique azoté saturé à 5 ou 6 chaînons qui peut avoir un groupe hydroxy, alkyle en C₁-C₈ ou hydroxyalkyle en C₁-C₈ ou oxo, dans lequel le groupe carbonyle ainsi formé peut être protégé par un groupement protecteur approprié ; pourvu que, lorsque l'un des restes R₄ et R₅ est l'hydrogène ou un groupe alkyle en C₁-C₈, l'autre soit toujours un groupe cyano ou ledit groupe alkyle en C₁-C₈ substitué et, lorsque R₄ et R₅ ne sont pas l'hydrogène ou un groupe alkyle inférieur, tous les deux soient choisis parmi un groupe cyanoet ledit groupe alkyle inférieur substitué,
ou bien R₄ est un atome d'hydrogène ou un groupe alkyle en C₁-C₈ et R₃ et R₅ sont combinés pour former un groupe de formule : 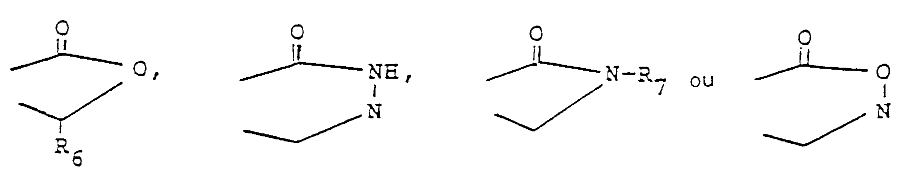 dans lesquelles R₆ est un atome d'hydrogène ou un groupe méthyle et R₇ est un groupe 2-(N,N-diéthylamino)éthyle ou 2-hydroxyéthyle, où le terme "inférieur" signifie 1 ou 2 à 8 atomes de carbone.

5. Un procédé tel que défini à la revendication 3, dans lequel l'énantiomère du composé chiral pharmacologiquement actif est la (+)-nilvadipine.

6. Un procédé tel que défini à la revendication 5, dans lequel le véhicule comprend un activateur de flux choisi parmi les esters d'acides gras en C₁₂-C₁₈ avec des alcools à chaîne droite ou ramifiée en C₁-C₆ ; les diesters de diacides de formule :
R₈OOC(CH₂)ₙ COO R₉
dans laquelle n est un entier de 2 à 8 et R₈ et R₉ peuvent être identiques ou différents et sont choisis parmi les alcools à chaîne droite et à chaîne ramifiée en C₂-C₁₂; les composés de formule : 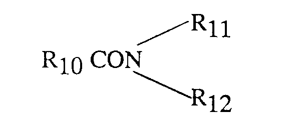 dans laquelle R₁₀ est un résidu à chaîne droite ou ramifiée d'un acide gras en C₈-C₁₄, R₁₁ et R₁₂ sont identiques ou différents et sont choisis parmi -CH₂CH₂OH et -CH₂CH₂CH₂OH; l'alcool benzylique, le 2-phényl-éthanol, l'éthanol ou les mélanges de ces solvants avec l'éthanol à 10-90 %.

7. Un procédé tel que défini à la revendication 5, dans lequel le solvant est l'adipate de diisopropyle.
